# EUROPEAN PATENT APPLICATION

(11) **EP 3 843 106 A1**
(43) Date of publication of application: **30.06.2021**
(21) Application number: 19853202.0
(22) Date of filing: 18.07.2019
(51) Int. Cl.: G16H 50/30, G16H 50/70, G16H 10/60

(54) **DEVICE AND METHOD FOR VISUALIZING VARIATION IN RISK OF DISEASES IN ACCORDANCE WITH CHANGES IN ENVIRONMENTAL FACTORS**

(30) Priority: 24.08.2018 KR 20180099044
(71) Applicant: Clinomics Inc., Ulsan 44919 (KR)
(72) Inventor: CHO, Yun Sung, Yongin-Si Gyeonggi-do 16953 (KR); JUN, Je Hoon, Suwon-Si Gyeonggi-do 16239 (KR); LEE, Hwang Yeol, Seoul 07264 (KR); KIM, Byung Chul, Suwon-Si Gyeonggi-do 16713 (KR); BHAK, Jong Hwa, Ulju-Gun Ulsan 44936 (KR)
(74) Representative: Altmann Stößel Dick Patentanwälte PartG mbB
(86) International application number: PCT/KR2019/008881
(87) International publication number: WO 2020/040431

(57) **Abstract**

The present invention relates to a device and a method for visualizing variation in the risk of diseases in accordance with changes in environmental factors which calculate a risk of diseases by maintaining a value with respect to genetic factors and changing a value with respect to environmental factors, among factors influencing the occurrence of disease, and visualize the risk of diseases calculated on the basis of the changes in environmental factors so that a user can numerically/visually ascertain how the environmental factors (lifestyle) should be improved or retained to lower the possibility of disease and can ultimately be led to make behavioral changes in order to more actively form better habits for preventing diseases.

## Description

### [Technical Field]

The present invention relates to a device and a method for visualizing variation in the risk of diseases in accordance with changes in environmental factors, and more particularly, to a device and a method which calculate a risk of diseases by retaining a value with respect to genetic factors and changing a value with respect to environmental factors, among factors influencing an occurrence of disease, and visualize the risk of diseases calculated on the basis of the changes in the environmental factors.

### [Background Art]

Various disease-related or phenotype-related gene mutation (SNP or mutation) markers are being revealed by genome sequence decoding and disease study (Genome-wide association study: GWAS). For example, it is reported that BRCA1 or BRCA2 gene mutations increase the possibility of diseases such as breast cancer.

Accordingly, various methods for predicting the possibility of occurrence of disease or non-disease phenotypes of a subject by utilizing various genetic markers reported through existing scientific researches have been reported (Cited Document: Schrodi SJ et al. Front. Genet. 2014) and domestic and foreign genetic testing institutions also predict/test the possibility of diseases on the basis of the calculation of the genetic risk of disease and phenotype by utilizing a similar method.

However, for the occurrence of the disease or phonotype, a lot of genetic factors and environmental factors (for example, a lifestyle) interact with each other and complexly affect. Accordingly, only when the risk of diseases is calculated by considering not only the possessed genetic factors, but also the environmental factors such as a lifestyle, exact prediction and analysis may be achieved.

The genetic factor which influences the disease cannot be improved by an individual (because it is difficult to improve without using a specific technique such as gene scissors), but in the case of the environmental factor which influences the disease, risk factors may be controlled by improving the lifestyle so that consequentially, the possibility of occurrence of disease may be improved. When a genetic test result is provided, if a degree of change in the risk of diseases in accordance with the change of current lifestyle (improved or deteriorated) is calculated to be presented, it is expected that a subject may numerically/visually ascertain how to improve or retain the environmental factors and ultimately and make behavioral changes to more actively form better habits for preventing the diseases.

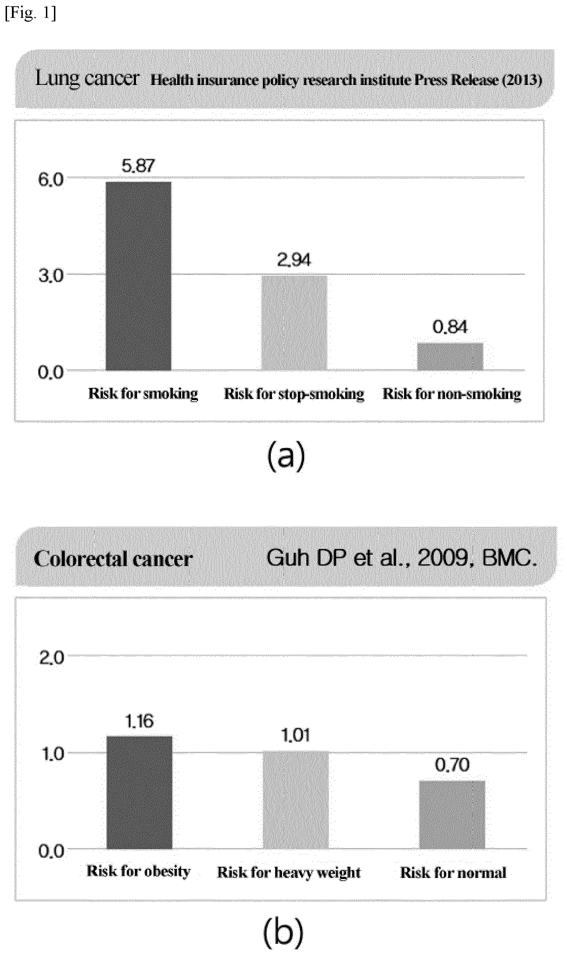

However, there has not been a technique to calculate how the risk of disease is changed when the environmental factors such as current lifestyle is changed (improved or deteriorated) and visualize the result. As illustrated in FIG. 1, there is a technique to show a changed value of risk in accordance with individual environmental factors with respect to two factors of "smoking" and "obesity", among various environmental factors. However, according to this technique, the risk is calculated first by considering only the genetic risk factor, without taking into account the current lifestyle of the subject, and then only a value of the risk which changes in accordance with the reported environmental factor states is shown. That is, referring to FIG. 1, my current state is not considered, but changes in the value, for all number of cases of environmental factors, which has been reported through the existing papers are simply applied to be presented. In the case of lung cancer, only when the genetic factor of the subject is considered, the risk is analyzed to be 0.84. Further, according to the epidemiological investigation which has been reported, it was reported that when a patient smokes, the risk is 6.99 times higher than that of non-smokers and when a patient smoked, but stopped smoking, the risk is increased by 3.5 times that of non-smokers. Therefore, the risk for the non-smoking case shows a value obtained by multiplying 0.84 of a genetic factor and 1 of an environmental factor in accordance with the non-smoking, the risk for a stopping-smoking case shows a value obtained by multiplying 0.84 of a genetic factor and 3.5 of an environmental factor in accordance with stop-smoking, and the risk for a smoking case shows a value obtained by multiplying 0.84 of a genetic factor and 6.99 of an environmental factor in accordance with smoking.

### [Disclosure]

### [Technical Problem]

A technical object to be achieved by the present invention is to provide a device and a method for visualizing variation in the risk of diseases in accordance with changes in environmental factors which calculate a risk of diseases by maintaining a value with respect to genetic factors and changing a value with respect to environmental factors, among factors influencing the occurrence of disease, and visualize the risk of diseases calculated on the basis of the changes in environmental factors.

### [Technical Solution]

In other to achieve the above-described technical object, according to the present invention, a device for visualizing variation in a risk of diseases in accordance with changes in environmental factors includes a storage unit which stores disease-causing factor information including genetic factors and environmental factors influencing occurrence of the disease, for every disease, a risk-of-disease calculating unit which calculates a current risk of diseases of the user with respect to a predetermined disease using a current value of the user for a genetic factor and an environmental factor influencing occurrence of the predetermined disease and calculates a changed risk of diseases of the user with respect to the predetermined disease using the current value of the user for a genetic factor influencing the occurrence of the predetermined disease and using a changed value on the basis of the current value for an environmental factor influencing the occurrence of the predetermined disease, on the basis of the disease-causing factor stored in the storage unit, and a display unit which visualizes and outputs the current risk of diseases and the changed risk of diseases of the user calculated by the risk-of-disease calculating unit.

When there is a plurality of environmental factors which influences the occurrence of the predetermined disease, the risk-of-disease calculating unit may calculate a changed risk of diseases of the user using a changed value on the basis of the current value of the user, for at least one of environmental factors, among the plurality of environmental factors.

The risk-of-disease calculating unit may calculate the changed risk of diseases of the user plural times by changing the changed value on the basis of the current value of the user, for the environmental factor influencing the occurrence of the predetermined disease and the display unit visualizes and outputs the current risk of diseases and the plurality of changed risks of diseases of the user calculated by the risk-of-disease calculating unit.

The changed value on the basis of the current value of the user may be a value improved on the basis of the current value of the user or a value deteriorated on the basis of the current value of the user.

When the display unit visualizes the current risk of diseases of the user and the plurality of changed risks of diseases, the display unit may visualize a ranking of the risk of the user, among all the users, together.

In order to achieve the above-described technical object, according to the present invention, a method for visualizing variation in a risk of diseases by a device of visualizing a variation in a risk of diseases includes calculating a current risk of diseases of the user with respect to a predetermined disease using a current value of the user for a genetic factor and an environmental factor influencing occurrence of the predetermined disease, on the basis of disease-causing factor information including genetic factors and environmental factors influencing the occurrence of a disease, for every disease; calculating a changed risk of diseases of the user with respect to the predetermined disease using the current value of the user for a genetic factor influencing the occurrence of the predetermined disease and using a changed value on the basis of the current value for an environmental factor influencing the occurrence of the predetermined disease, on the basis of the disease-causing factor information; and visualizing and outputting the calculated current risk of diseases and changed risk of diseases of the user.

In the calculating of a changed risk of diseases, when there is a plurality of environmental factors which influences the occurrence of the predetermined disease, a changed risk of diseases may be calculated using a changed value on the basis of the current value of the user, for at least one of environmental factors, among the plurality of environmental factors.

In the calculating of a changed risk of diseases, the changed risk of diseases of the user may be calculated plural times by changing a changed value on the basis of the current value of the user, for the environmental factor influencing the occurrence of the predetermined disease and in the outputting, the calculated current risk of diseases of the user and a plurality of changed risks of diseases may be visualized to be output.

The changed value on the basis of the current value of the user may be a value improved on the basis of the current value of the user or a value deteriorated on the basis of the current value of the user.

In the outputting, when the current risk of diseases of the user and the plurality of changed risks of diseases are visualized, a ranking of the risk of the user, among all the users, may also be visualized together.

To achieve the aforementioned technical object, a computer program according to the present invention is stored in a computer-readable recording medium to execute any one of the aforementioned methods on a computer.

### [Advantageous Effects]

According to the present invention, the device and method for visualizing variation in the risk of diseases in accordance with changes in environmental factors calculate a risk of diseases by maintaining a value with respect to genetic factors and changing a value with respect to environmental factors, among factors influencing the occurrence of disease, and visualize the risk of diseases calculated on the basis of the changes in environmental factors so that a user can numerically/visually ascertain how the environmental factors (lifestyle) should be improved or retained to lower the possibility of disease and can ultimately be led to make behavioral changes in order to more actively form better habits for preventing diseases.

### [Description of Drawings]

FIG. 1 is a view for explaining an example of visualization of a risk of diseases of the related art.
FIG. 2 is a block diagram for explaining a device for visualizing variation in the risk of diseases in accordance with changes in environmental factors according to an exemplary embodiment of the present invention.
FIG. 3 is a view for explaining an example of visualization of the risk of diseases according to an exemplary embodiment of the present invention.
FIG. 4 is a view for explaining another example of visualization of the risk of diseases according to an exemplary embodiment of the present invention.
FIG. 5 is a flowchart for explaining a method for visualizing variation in the risk of diseases in accordance with changes in environmental factors according to an exemplary embodiment of the present invention.

### [Modes of the Invention]

Hereinafter, exemplary embodiments of a device and a method for visualizing variation in the risk of diseases in accordance with changes in environmental factors according to the present invention will be described in detail with reference to the accompanying drawings.

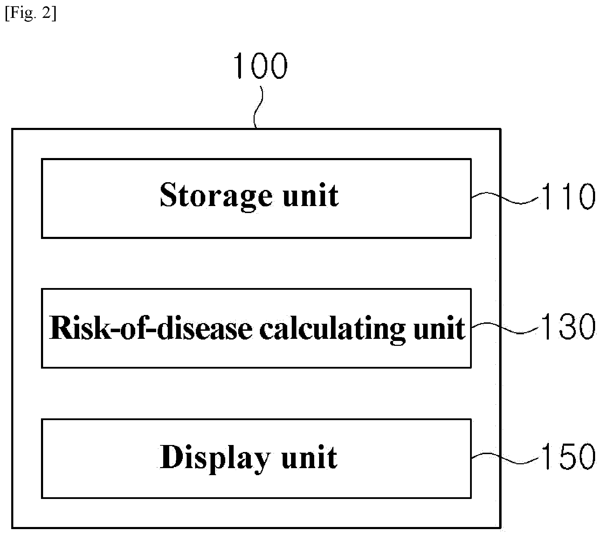

First, a device for visualizing variation in the risk of diseases in accordance with changes in environmental factors according to an exemplary embodiment of the present invention will be described with reference to FIGS. 2 to 4.

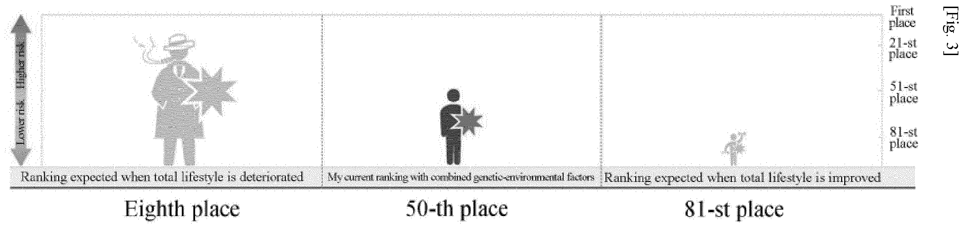

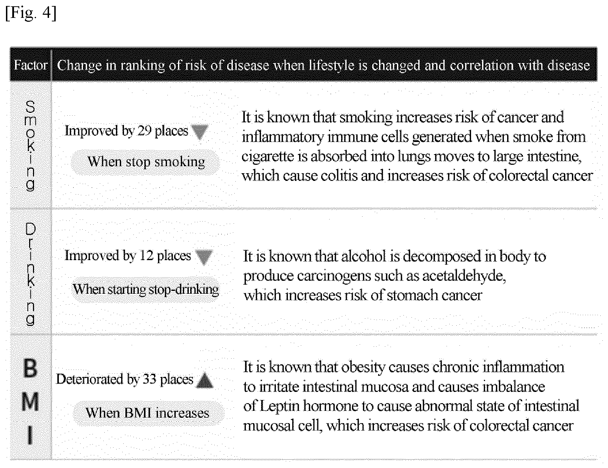

FIG. 2 is a block diagram for explaining a device for visualizing variation in the risk of diseases in accordance with changes in environmental factors according to an exemplary embodiment of the present invention, FIG. 3 is a view for explaining an example of visualization of the risk of diseases according to an exemplary embodiment of the present invention, and FIG. 4 is a view for explaining another example of visualization of the risk of diseases according to an exemplary embodiment of the present invention.

Referring to FIG. 2, a device 100 for visualizing variation in the risk of diseases in accordance with the changes in environmental factors according to an exemplary embodiment of the present invention calculates a risk of diseases by retaining a value with respect to genetic factors and changing a value with respect to environmental factors, among factors influencing an occurrence of diseases and visualizes the risk of diseases calculated on the basis of the changes in the environmental factors.

To this end, the device 100 for visualizing variation in the risk of diseases may include a storage unit 110, a risk-of-disease calculating unit 130, and a display unit 150.

The storage unit 110 stores disease-causing factor information. Here, the disease-causing factor information includes information for every disease, about genetic factors and environmental factors which influence an occurrence of the diseases.

For example, the disease-causing factor information may be acquired by collecting genetic markers (genetic factors) with respect to diseases (or phenotype) and environmental markers (environmental factors) from existing materials such as papers together with effect sizes of each marker (factor). Generally, the effect size of the marker with respect to a disease may be calculated by one of an odds ratio, a relative risk, a risk ratio, and a hazard ratio.

The risk-of-disease calculating unit 130 calculates a current risk of diseases and a changed risk of diseases of a user with respect to predetermined diseases, on the basis of disease-causing factor information stored in the storage unit 110. Here, in order to calculate the current risk of diseases or the changed risk of diseases, various existing methods may be used.

That is, the risk-of-disease calculating unit 130 may calculate the current risk of diseases of the user for a predetermined disease using a current value of the user for the genetic factor and the environmental factor which influence the occurrence of the predetermined disease, on the basis of the disease-causing factor information.

For example, referring to the following Table 1, if environmental factors influencing occurrence of stomach cancer are drinking, smoking, and a body mass index (BMI) and the current value (current lifestyle) of the user for the environmental factor (lifestyle) is as represented in the following Table 1, the current risk of diseases of the user with respect to the stomach cancer takes the "fiftieth place". The risk of diseases according to the following Table 1 is just an example and the risk of diseases is calculated by various existing calculating methods and thus may be represented by various methods such as scores, a multiple, a rank, or a level.

**[Table 1]**

| Environmental factor | Value (Lifestyle) | Effect size | User's current value (Current lifestyle) | User's risk of disease |
|---|---|---|---|---|
| Drinking | Heavy drinking | 1.26 times | | Fiftieth place / 100 person (one example of indicating risk by representing place out of 100 people / the closer to the first place, the larger the risk) |
| | Appropriate drinking | 1.06 times | ○ | |
| | Little or not drinking | 1 time | | |
| Smoking | Smoking | 1.56 times | ○ | |
| | Non-smoking | 1 time | | |
| BMI | Obesity | 1.41 times | | |
| | Overweight | 1.10 times | ○ | |
| | Normal weight | 1 time | | |

Further, the risk-of-disease calculating unit 130 may calculate a changed risk of diseases of the user with respect to the predetermined disease using a current value of the user for the genetic factor influencing the occurrence of a predetermined disease and using a changed value on the basis of the current value of the user for the environmental factor influencing the occurrence of the predetermined disease, on the basis of the disease-causing factor information. That is, the risk-of-disease calculating unit 130 may calculate the changed risk of diseases by retaining a value for the genetic factor, but changing a value for the environmental factor, among factors influencing the occurrence of the disease. Here, the changed value on the basis of the current value of the user may be a value improved from the current value of the user or a value deteriorated from the current value of the user.

In this case, when there is a plurality of environmental factors which influences the occurrence of the predetermined disease, the risk-of-disease calculating unit 130 may calculate a changed risk of diseases of the user using the value changed on the basis of the current value of the user, with respect to at least one of environmental factors, among the plurality of environmental factors. For example, if there are three environmental factors, the risk-of-disease calculating unit 130 may calculate the changed risk of diseases of the user using the changed value with respect to one environmental factor or two or three environmental factors.

Further, the risk-of-disease calculating unit 130 may calculate the changed risk of diseases of the user plural times by changing a changed value on the basis the current value of the user, with respect to the environmental factor influencing the occurrence of the predetermined disease. For example, the changed risk of diseases of the user is calculated using "A" as the current value of the user with respect to the environmental factor, the changed risk of diseases of the user is calculated again using "B" as the current value of the user with respect to the environmental factor, and the changed risk of diseases of the user may be calculated again using "C" as the current value of the user with respect to the environmental factor.

For example, referring to the following Table 2, the environmental factor influencing the occurrence of the stomach cancer is drinking, smoking, and BMI (body mass index) and the changed risk of diseases of the user with respect to the stomach cancer may be calculated by changing (improving or deteriorating) the current value (current lifestyle) of the user with respect to the environmental factor. The changed degree of the value for the environmental factor according to the following Table 2 is just an example, and the changed degree may be set in various manners.

**[Table 2]**

| Environmental factor | Value (Lifestyle) | Example 1 (Drinking is improved) | Example 2 (BMI is deteriorated) | Example 3 (Two factors are improved) | Example 4 (Improved to maximum) |
|---|---|---|---|---|---|
| Drinking | Heavy dinking | | | | |
| | Appropriate drinking | | ○ | | |
| | Little or not drinking | ○ (Improved) | | ○ (Improved) | ○ (Improved) |
| Smoking | Smoking | ○ | ○ | | |
| | Non-smoking | | | ○ (Improved) | ○ (Improved) |
| BMI | Obesity | | ○ (Deteriorated) | | |
| | Overweight | ○ | | ○ | |
| | Normal weight | | | | ○ (Improved) |
| User's risk of disease according to change in environmental factor | | 62-nd place (Improved by 12 places) | 17-th place (Deteriorated by 33 places) | 75-th place (Improved by 25 places) | 81-st place (Improved by 31 places) |

The display unit 150 visualizes and outputs the current risk of diseases and the changed risk of diseases of the user calculated by the risk-of-disease calculating unit 130.

Further, when the changed risk of diseases is calculated plural times by the risk-of-disease calculating unit 130, the display unit 150 may visualize and output the current risk of diseases of the user calculated by the risk-of-disease calculating unit 130 and a plurality of changed risks of diseases.

At this time, when the display unit 150 visualizes the current risk of diseases of the user and the plurality of changed risks of diseases, the display unit 150 may visualize a ranking of the risk of the current user among all the users together.

For example, as illustrated in FIG. 3, not only the ranking of the user for a specific disease according to the current risk of diseases is displayed, but also how much the risk of diseases which may be improved/deteriorated according to the change of the environmental factor (lifestyle) is changed may be displayed. In addition, as illustrated in FIG. 4, a change in the ranking of the risk of diseases expected when an environmental factor (lifestyle) is changed is displayed to allow the user to specifically ascertain which environmental factor (lifestyle) needs to be improved and how the risk of diseases is changed when the current good environmental factor (lifestyle) is deteriorated.

In the meantime, although the device 100 for visualizing variation in the risk of diseases according to an exemplary embodiment of the present invention has been described as one independent device, that is, the device 100 for visualizing variation in the risk of diseases calculates the current risk of diseases of the user, calculates a changed risk of diseases in accordance with the change in the environmental factor, and visualizes and outputs the calculated current risk of diseases and changed risk of diseases, it is not limited thereto. According to the exemplary embodiment, the device 100 for visualizing variation in the risk of diseases according to the present invention may be installed in a user terminal (not illustrated) such as a smart phone or a notebook as an application to perform the operation according to the present invention.

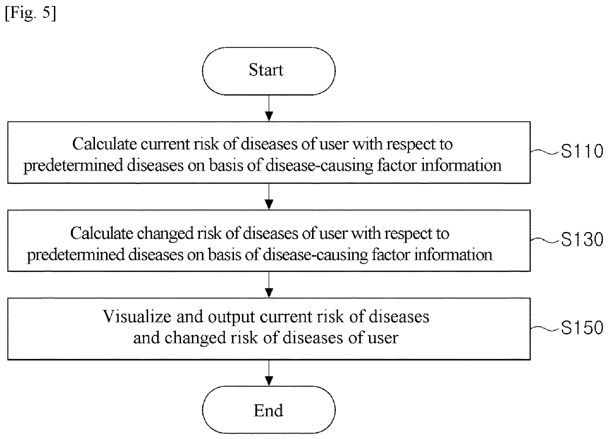

Now, a method for visualizing variation in the risk of diseases on the basis of changes in environmental factors according to an exemplary embodiment of the present invention will be described with reference to FIG. 5.

FIG. 5 is a flowchart for explaining a method for visualizing variation in the risk of diseases in accordance with changes in environmental factors according to an exemplary embodiment of the present invention.

Referring to FIG. 5, the device 100 for visualizing variation in the risk of diseases calculates a current risk of diseases of the user with respect to a predetermined disease on the basis of disease-causing factor information (S110). Here, the disease-causing factor information includes information for every disease, about genetic factors and environmental factors which influence the occurrence of the diseases.

That is, the device 100 for visualizing variation in the risk of diseases may calculate the current risk of diseases of the user with respect to a predetermined disease using a current value of the user for the genetic factor and the environmental factor which influence the occurrence of the predetermined disease, on the basis of the disease-causing factor information.

Further, the device 100 for visualizing variation in the risk of diseases calculates a changed risk of diseases of the user with respect to a predetermined disease on the basis of disease-causing factor information (S130).

That is, the device 100 for visualizing variation in the risk of diseases may calculate a changed risk of diseases of the user with respect to the predetermined disease using a current value of the user for the genetic factor influencing the occurrence of a predetermined disease and using a changed value on the basis of the current value of the user for the environmental factor influencing the occurrence of the predetermined disease, on the basis of the disease-causing factor information. Here, the changed value on the basis of the current value of the user may be a value improved from the current value of the user or a value deteriorated from the current value of the user.

In this case, when there is a plurality of environmental factors which influences the occurrence of the predetermined disease, the device 100 for visualizing variation in the risk of diseases may calculate a changed risk of diseases of the user using the changed value on the basis of the current value of the user, for at least one of environmental factors, among the plurality of environmental factors.

Further, the device 100 for visualizing variation in the risk of diseases calculates the changed risk of diseases of the user plural times by changing a value changed on the basis of the current value of the user, for the environmental factor influencing the occurrence of the predetermined disease.

Next, the device 100 for visualizing variation in the risk of diseases visualizes and outputs the current risk of diseases and the changed risk of diseases of the user (S150).

When the changed risk of diseases is calculated plural times, the device 100 for visualizing variation in the risk of diseases may visualize and output the calculated current risk of diseases of the user and a plurality of changed risks of diseases.

At this time, when the device 100 for visualizing variation in the risk of diseases visualizes the current risk of diseases of the user and the plurality of changed risks of diseases, the device 100 for visualizing variation in the risk of diseases may visualize a ranking of the risk of the corresponding user, among all the users, together.

The present disclosure may also be implemented as computer-readable codes written on a computer-readable recording medium. The computer-readable recording medium includes all types of recording devices on which data may be recorded in a computer-readable manner. Examples of the computer readable recording medium includes a ROM, a RAM, a CD-ROM, a magnetic tape, a floppy tape, an optical data storage device, and the like.

Although the exemplary embodiment of the present disclosure has been described in detail above, the present disclosure is not limited to the specific exemplary embodiment of the present disclosure, and a person of ordinary skill in the art may variously modify and work the present disclosure without departing from the principals of the present disclosure defined in the claims, and the modification belongs to the scope of the claims.

### [Explanation of Reference Numerals and Symbols]

100: Device for visualizing variation in risk of diseases
110: Storage unit
130: Risk-of-disease calculating unit
150: Display unit

## Claims

1. A device for visualizing variation in a risk of diseases in accordance with changes in environmental factors, the device comprising:
a storage unit which stores disease-causing factor information including genetic factors and environmental factors influencing occurrence of the disease, for every disease;
a risk-of-disease calculating unit which calculates a current risk of diseases of the user with respect to a predetermined disease using a current value of the user for a genetic factor and an environmental factor influencing occurrence of the predetermined disease and calculates a changed risk of diseases of the user with respect to the predetermined disease using the current value of the user for a genetic factor influencing the occurrence of the predetermined disease and using a changed value on the basis of the current value of the user for an environmental factor influencing the occurrence of the predetermined disease, on the basis of the disease-causing factor stored in the storage unit; and
a display unit which visualizes and outputs the current risk of diseases and the changed risk of diseases of the user calculated by the risk-of-disease calculating unit.

2. The device of claim 1, wherein when there is a plurality of environmental factors which influences the occurrence of the predetermined disease, the risk-of-disease calculating unit calculates a changed risk of diseases of the user using a changed value on the basis of the current value of the user, for at least one of environmental factors, among the plurality of environmental factors.

3. The device of claim 1, wherein the risk-of-disease calculating unit calculates the changed risk of diseases of the user plural times by changing the changed value on the basis of the current value of the user, for the environmental factor influencing the occurrence of the predetermined disease and the display unit visualizes and outputs the current risk of diseases and the plurality of changed risks of diseases of the user calculated by the risk-of-disease calculating unit.

4. The device of claim 1, wherein the changed value on the basis of the current value of the user is a value improved on the basis of the current value of the user or a value deteriorated on the basis of the current value of the user.

5. The device of claim 1, wherein when the display unit visualizes the current risk of diseases of the user and the plurality of changed risks of diseases, the display unit visualizes a ranking of the risk of the user, among all the users, together.

6. A method for visualizing variation in a risk of diseases by a device of visualizing a variation in a risk of diseases in accordance with changes in environmental factors, the method comprising:
calculating a current risk of diseases of the user with respect to a predetermined disease using a current value of the user for a genetic factor and an environmental factor influencing occurrence of the predetermined disease, on the basis of disease-causing factor information including genetic factors and environmental factors influencing the occurrence of a disease, for every disease;
calculating a changed risk of diseases of the user with respect to the predetermined disease using the current value of the user for a genetic factor influencing the occurrence of the predetermined disease and using a changed value on the basis of the current value of the user for an environmental factor influencing the occurrence of the predetermined disease, on the basis of the disease-causing factor information; and
visualizing and outputting the calculated current risk of diseases and changed risk of diseases of the user.

7. The method of claim 6, wherein in the calculating of a changed risk of diseases, when there is a plurality of environmental factors which influences the occurrence of the predetermined disease, a changed risk of diseases of the user is calculated using a changed value on the basis of the current value of the user, for at least one of environmental factors, among the plurality of environmental factors.

8. The method of claim 6, wherein in the calculating of a changed risk of diseases, the changed risk of diseases of the user is calculated plural times by changing a changed value on the basis of the current value of the user, for the environmental factor influencing the occurrence of the predetermined disease and
in the outputting, the calculated current risk of diseases of the user and a plurality of changed risks of diseases are visualized to be output.

9. The method of claim 6, wherein the changed value on the basis of the current value of the user is a value improved on the basis of the current value of the user or a value deteriorated on the basis of the current value of the user.

10. The method of claim 6, wherein in the outputting, when the current risk of diseases of the user and the plurality of changed risks of diseases are visualized, a ranking of the risk of the user, among all the users, is visualized together.

11. A computer program stored in a computer readable recording medium to cause a computer to execute the method for visualizing variation in a risk of diseases in accordance with changes in environmental factor of any one of claims 6 to 10.
